# EUROPÄISCHE PATENTSCHRIFT

(11) **EP 3 202 427 B1**
(45) Veröffentlichungstag und Bekanntmachung des Hinweises auf die Patenterteilung: **03.04.2019**
(21) Anmeldenummer: 16154097.6
(22) Anmeldetag: 03.02.2016
(51) Int. Cl.: A61L 27/04

(54) **VERWENDUNG EINER AUSSCHEIDUNGSHÄRTENDEN ODER DURCH MISCHKRISTALLBILDUNG VERFESTIGENDEN, BIOKOMPATIBLEN KOBALT-BASISLEGIERUNG UND VERFAHREN ZUR HERSTELLUNG VON IMPLANTATEN ODER PROTHESEN DURCH MATERIALABHEBENDE BEARBEITUNG**
USE OF A BIOCOMPATIBLE COBALT BASED ALLOY HARDENED BY PRECIPITATION OR REINFORCING BY MIXED CRYSTAL FORMING AND METHOD FOR THE MANUFACTURE OF IMPLANTS OR PROSTHETICS BY MATERIAL REMOVAL
UTILISATION D'UN ALLIAGE À BASE DE COBALT BIOCOMPATIBLE, SE SOLIDIFIANT PAR FORMATION DE CRISTAUX MIXTES OU TREMPÉ PAR PRÉCIPITATION ET PROCÉDÉ DE FABRICATION D'IMPLANTS OU DE PROTHÈSES PAR ENLEVEMENT DE MATIÈRE

(43) Veröffentlichungstag der Anmeldung: 09.08.2017
(73) Patentinhaber: Deutsche Edelstahlwerke Specialty Steel GmbH & Co. KG, 58452 Witten (DE)
(72) Erfinder: van Bennekom, André, 57234 Wilnsdorf (DE); Hill, Horst, 47929 Grefrath (DE); Ripkens, Oliver, 47647 Kerken (DE)
(74) Vertreter: Cohausz & Florack

(56) Entgegenhaltungen:
- EP-A1- 0 257 262
- EP-A1- 1 657 318
- WO-A2-2005/007909
- DE-A1- 2 621 789
- DE-A1- 19 704 530
- DE-C1- 4 229 599
- DATABASE WPI Week 200921 Thomson Scientific, London, GB; AN 2009-F56907 XP002759658, & JP 2009 046760 A (KOBE STEEL LTD) 5. März 2009 (2009-03-05) -& DATABASE EPODOC [Online] EUROPEAN PATENT OFFICE, THE HAGUE, NL; XP002759659, Database accession no. JP-2008168978-A

## Beschreibung

Die Erfindung betrifft eine Verwendung einer ausscheidungshärtenden oder durch Mischkristallbildung verfestigenden, biokompatiblen Kobalt-Basislegierung und ein Verfahren zur Herstellung von Implantaten durch materialabhebende Bearbeitung.

Wenn im vorliegenden Text Angaben zu Gehalten von Legierungen gemacht werden, beziehen sich diese immer auf das Gewicht, solange nichts anderes ausdrücklich angegeben ist.

Für die Herstellung von Bauteilen, die in oder am menschlichen oder tierischen Körper verwendet werden sollen, wird ein breites Werkstoffspektrum eingesetzt. Dieses erstreckt sich von den Kunststoffen über Keramikwerkstoffe und Edelmetalwerkstoffe bis hin zu metallischen Werkstoffen, die aus Nichtedelmetallen bestehen.

Die Bezeichnung "Bauteil zur Verwendung in oder am menschlichen oder tierischen Körper" umfasst hier sowohl Implantate, die fest im Körper verbaut werden, wie Schrauben, Schienen, Spangen, Teile von Hüft- oder Kniegelenken, Zahnpfeiler oder andere fest im Kiefer verankerte Zahnimplantate und andere als Ersatz für natürliche Knochen oder Gelenke implantierte Teile, als auch Prothesen, die vorübergehend oder dauerhaft am Körper befestigt werden, wie Teile der Zahnprothetik (Brücken, Zahnteil- oder -vollersatz) oder Werkzeuge, die insbesondere bei der Zahnbehandlung benötigt werden. Diese auf unedlen Metallen basierenden Werkstoffe umfassen vor allem Legierungen auf Co- oder Ni-Basis, wie CoCr- und NiCr-Werkstoffe, welche typischerweise für zahntechnische Anwendungen, aber auch für Gelenkimplantate, insbesondere Hüft- oder Knieimplantate, zur Anwendung kommen.

Werkstoffe für Implantate oder Prothesen müssen ausreichend korrosionsbeständig sein und eine optimierte Bioverträglichkeit aufweisen. Sie dürfen folglich im Gebrauch keine schädlichen Auswirkungen auf den Körper haben, in den oder an dem die aus ihnen gefertigten Bauteile eingesetzt werden, und auch keine sonstige Reaktionen auslösen, die einen ungünstigen Einfluss auf das Wohlbefinden oder die Gesundheit haben könnten.

Gleichzeitig müssen Implantat- oder Prothesenwerkstoffe eine für den jeweiligen Verwendungszweck ausreichende mechanische Eigenschaft, wie Festigkeit, Zähigkeit und desgleichen, besitzen.

Schließlich müssen sich derartige Werkstoffe auch gut be- und verarbeiten lassen, um die in vielen Fällen besonders filigrane und jeweils individuell angepasste Gestaltung von Implantaten oder Prothesen verwirklichen zu können.

Im Falle von Werkstoffen auf Kobaltbasis der hier in Rede stehenden Art sind im Bereich der Dentaltechnik drei Fertigungsrouten etabliert, die jeweils unterschiedliche Anforderungen an das Werkstoffverhalten mit sich bringen.

Hierzu zählen konventionelle Gussverfahren, bei denen anhand eines Modells das jeweilige Implantat oder die jeweilige Prothese abgebildet wird. Beim Beispiel eines im Mundraum zum Einsatz kommenden Implantats wird hierzu zunächst mittels einer Abdruckmasse eine Negativform des Mundraumes erzeugt. Darauf aufbauend wird eine verlorene Form erzeugt, in die die Metallschmelze gegossen wird. Im Anschluss daran folgt in der Regel eine mechanische Nachbearbeitung, um eine optimale Anpassung der Form des Implantats zu gewährleisten. Handelt es sich bei dem Implantat oder der Prothese um einen Zahnersatz, so wird der nach der Implantierung oder Anordnung im Mundraum sichtbare Teil üblicherweise mit einer Keramikverblendung versehen. In diesem Fall muss der Metallwerkstoff des Implantats oder des Implantats nicht nur optimierte mechanische Eigenschaften und eine ebenso optimierte Kompatibilität, sondern auch noch eine optimierte Eignung für den in der Regel durch Einbrennen erfolgenden Auftrag der Keramikverblendung besitzen. Als Zahnersatz dienende, dauerhaft im Mundraum verbleibende Prothesen werden dabei üblicherweise an einem in den Kiefer implantierten Pfeiler oder einem noch vorhandenen Zahn befestigt.

Alternativ zu den konventionellen Gussverfahren sind auch so genannte additive Fertigungsverfahren für die Herstellung von Implantaten bekannt. Diese basieren in der Regel auf Metallpulvern und kommen ebenfalls besonders bei der Herstellung von Prothesen oder Implantaten zum Einsatz, die im Mundraum eingesetzt werden. Zu den additiven Verfahren zählen allgemein der 3D-Druck und speziell das Lasersintern oder Laserschmelzen. Bei den additiven Verfahren wird vom Mundraum in der Regel ein digitales Abbild erstellt und daraus ein 3D-Modell des herzustellenden Implantats erzeugt. Beim Lasersintern wird dann ein Legierungspulver entsprechend dem 3D-Modell schichtweise aufgetragen und mittels eines Laserstrahls versintert bzw. aufgeschmolzen. Der Vorteil solcher additiver Verfahren besteht darin, dass komplex geformte Implantate ohne Rücksichtig auf die bei konventionellen Gussverfahren bestehende Problematik der Entformung verwirklicht werden können und es gleichzeitig möglich ist, individuell geformte Implantatbauteile im industriellen Maßstab zu produzieren. Der Stand der Technik zum 3D-Druck von Zahnersatz ist im Artikel "3D printing of dental restorations", metalpowder Report, No 2, March/April 2013, Seiten 32,33, zusammengefasst, wogegen ein Beispiel für ein Verfahren zur Herstellung von Zahnimplantaten durch Lasersintern in der EP 1 972 321 B1 beschrieben ist.

Als drittes im Stand der Technik etabliertes Verfahren zur Herstellung insbesondere von Implantaten oder Prothesen, die im Dentalbereich eingesetzt werden, sind die so genannten subtraktiven oder abtragenden Verfahren zu nennen. Bei diesen Verfahren wird ein aus dem jeweiligen Implantatwerkstoff bestehender Rohling bereitgestellt, aus dem anschließend mittels spanabhebender Bearbeitung das jeweilige Implantat herausgearbeitet wird. Auch für diese Verfahren wird zunächst ein digitales Abbild des Mundraums bzw. des Bereichs, in den das Implantat eingesetzt werden soll, und aus diesem Abbild ein 3D-Modell des Implantats erzeugt. Anschließend wird das Implantat "aus dem Vollen" in der Regel durch Fräsbearbeitung des Rohlings gefertigt. Abhängig von der Geometrie des herzustellenden Implantats kommen dabei als Rohlinge Scheiben, Würfel, Zylinder oder Quader zum Einsatz.

Um eine Zulassung für die Verwendung als Implantatwerkstoff zu erhalten, müssen diese Werkstoffe umfangreiche Untersuchungen durchlaufen, bei denen insbesondere die Biokompatibilität und Dauerhaltbarkeit nachgewiesen werden muss. Wegen des mit diesen Prüfungen verbundenen Aufwands besteht ein grundsätzliches Bestreben, für die drei etablierten Fertigungsrouten gleiche Werkstoffe einzusetzen. Dementsprechend wird von den Implantatwerkstoffen ein breites Eigenschaftsspektrum verlangt.

Ein Charakteristikum von konventionellen für die Anwendung speziell im Mundraum vorgesehenen CoCr- und NiCr-Werkstoffen ist, dass sie naturhart sind. Das heißt, dass zur Einstellung ihrer Endhärte und sonstigen mechanischen Eigenschaften keiner speziellen Wärmebehandlung bedarf, sondern, dass das geforderte Eigenschaftsprofil unmittelbar nach der Erzeugung der jeweiligen Legierung vorliegt. Für die Fertigungsrouten I (konventionelles Gussverfahren) und II (additive Verfahren) stellt dies kein Problem dar, sondern ist in der Regel sogar gewünscht, da nach der formgebenden Herstellung die jeweilige Implantatkomponente allenfalls noch einer Feinbearbeitung, wie einem Schleifen oder Polieren, unterzogen werden müssen und es somit günstig ist, wenn die Zieleigenschaften schon im Material vorliegen.

Konventionelle kobaltbasierte Legierungen für Implantate erreichen, abhängig von ihrer Zusammensetzung im naturharten Zustand, hohe Festigkeiten (Streckgrenze > 500 MPa), wodurch eine Härte von bis zu 40 HRC entsteht.

Diese, an sich für den hier in Rede stehenden Anwendungszweck vorteilhaften Eigenschaften stehen bei den subtraktiven Bearbeitungsverfahren dem Materialabtrag entgegen. Die hohe Härte führt bei der spanabhebenden Bearbeitung des Rohlings zu erhöhtem Werkzeugverschleiß und verursacht eine verlängerte Bearbeitungsdauer.

DE19704530 A1 beschreibt Kobalt-Legierungen als kalt umformbarer Werkstoff für Zahnprothesen und Implantaten, dessen mechanische Eigenschaften durch Wärmebehandlung gesteigert werden können.

Vor dem Hintergrund des voranstehend erläuterten Standes der Technik hat sich die Aufgabe ergeben, Möglichkeiten aufzuzeigen, die eine vereinfachte Herstellung von Implantaten oder Prothesen, speziell von Dentalimplantaten oder -prothesen, durch Anwendung subtraktiver Verfahren erlauben.

Zur Lösung dieser Aufgabe hat die Erfindung die in Anspruch 1 angegebene Verwendung und das in Anspruch 2 angegebene Verfahren vorgeschlagen. Vorteilhafte Ausgestaltungen der Erfindung sind in den abhängigen Ansprüchen angegeben und werden nachfolgend, wie der allgemeine Erfindungsgedanke, im Einzelnen erläutert.

Die Erfindung schlägt dementsprechend die Verwendung einer ausscheidungshärtenden oder durch Mischkristallbildung verfestigenden, biokompatiblen Kobalt-Basislegierung für die Herstellung von Rohlingen vor, die dazu vorgesehen sind, dass aus ihnen im weichen Zustand durch spanabhebende Bearbeitung Implantate oder Prothesen herausgearbeitet werden, welche nach der spanabhebenden Bearbeitung einer Wärmebehandlung unterzogen werden, um die Endhärte der Implantate oder Prothesen durch Ausscheidungshärtung oder Mischkristallbildung einzustellen, wobei die Kobalt-Basislegierung aus (in Gew.-%) C: bis zu 0,1 %, Si: 0,8 - 2,0 %, Cr: 22 - 29 %, Mn: bis zu 1 %, Ni: bis zu 3 %, Fe: bis zu 3 %, Ce: bis zu 1 %, Ga: bis zu 5 %, B: bis zu 2,5 %, sowie jeweils mindestens einem der härtesteigernde Ausscheidungen oder Mischkristalle bildenden Elemente aus der Gruppe "Mo, W, Nb, Al, Ti", wobei für die Gehalte dieser Elemente, sofern vorhanden, die Maßgabe gilt: Mo: 3 - 9 %, W: 3 - 9 %, Nb: 3 - 9 %, Al: 0,1 - 6 %, Ti: 0,1 - 6 %, und als Rest Co und herstellungsbedingt unvermeidbare Verunreinigungen besteht.

Genauso schlägt die Erfindung vor, zum Herstellen eines Implantats, mindestens folgende Arbeitsschritte auszuführen:
- Bereitstellen eines weichen Rohlings, der aus einer ausscheidungshärtenden oder durch Mischkristallbildung verfestigenden, biokompatiblen Legierung auf Basis von Kobalt besteht,
- Herausarbeiten eines Implantats oder der Prothese aus dem weichen Rohling durch spanabhebende Bearbeitung,
- Wärmebehandeln des Implantats oder der Prothese zur Einstellung der Härte durch Ausscheidungshärtung oder Mischkristallbildung, wobei die Kobalt-Basislegierung aus (in Gew.-%) C: bis zu 0,1 %, Si: 0,8 - 2,0 %, Cr: 22 - 29 %, Mn: bis zu 1 %, Ni: bis zu 3 %, Fe: bis zu 3 %, Ce: bis zu 1 %, Ga: bis zu 5 %, B: bis zu 2,5 %, sowie jeweils mindestens einem der härtesteigernde Ausscheidungen oder Mischkristalle bildenden Elemente aus der Gruppe "Mo, W, Nb, Al, Ti", wobei für die Gehalte dieser Elemente, sofern vorhanden, die Maßgabe gilt: Mo: 3 - 9 %, W: 3 - 9 %, Nb: 3 - 9 %, Al: 0,1 - 6 %, Ti: 0,1 - 6 %, und als Rest Co und herstellungsbedingt unvermeidbare Verunreinigungen besteht.

Der erfindungsgemäßen Verwendung und dem erfindungsgemäßen Verfahren gemeinsam ist somit, dass der jeweilige Rohling, aus dem das jeweilige Implantat- oder Prothesenbauteil gefertigt werden soll, aus einem ausscheidungshärtenden oder durch Mischkristallbildung verfestigenden Co-Basiswerkstoff hergestellt ist, der im Ausgangszustand, d.h. dem Zustand, in dem er für die materialabtragende Bearbeitung bereitgestellt wird, noch nicht seine Endhärte erreicht hat, sondern so ausgelegt ist, dass er erst nach einer abschließenden Wärmebehandlung in Folge der dabei, sich im Gefüge des Werkstoffs bildenden Ausscheidungen, seine Endhärte erreicht.

Das weiche, durch eine geringe Härte gekennzeichnete Gefüge des erfindungsgemäß bereitgestellten Rohlings kann dabei insbesondere dadurch eingestellt werden, dass der Rohling vor der materialabhebenden Bearbeitung einem Lösungsglühen unterzogen wird, bei dem eine Härtereduzierung durch Auflösung von intermetallischen Phasen und Kornwachstum bewirkt wird.

Die Herstellung des Rohlings kann dabei in konventioneller Weise gießtechnisch oder durch Anwendung pulvermetallurgischer, ebenfalls an sich bekannter Verfahren erfolgen. Beispielhaft sind Gießen, Gießen und Umformen, Feinguss, Pulver- und Heiß-Isostatisches-Pressen (HIP) zu nennen.

Aufgrund seiner gegenüber der Endhärte des jeweiligen Bauteils geringen Härte lässt sich der erfindungsgemäß bereitgestellte Rohling mit geringerem Werkzeugverschleiß und erhöhter Geschwindigkeit mechanisch bearbeiten. Der Materialabtrag erfolgt dabei typischerweise durch spanabhebende Verfahren, insbesondere durch eine Fräsbearbeitung. Es sind jedoch auch alle anderen Verfahren zu diesem Zweck geeignet, die einen effektiven Materialabtrag unter Ausnutzung der bei einem erfindungsgemäß bereitgestellten Rohling vorhandenen geringen Härte ermöglichen.

Die vom fertigen Implantat- oder Prothesenbauteil geforderten mechanischen Eigenschaften werden gemäß der Erfindung durch eine Wärmebehandlung eingestellt, die nach der materialabtragenden, formgebenden Bearbeitung durchgeführt wird. Durch diese Wärmebehandlung wird die Ausscheidungshärtung oder Mischkristallbildung bewirkt und damit einhergehend die hohen Festigkeiten eingestellt, die Implantate insbesondere für den Dentalbereich aufweisen sollen. Ausscheidungshärtung und Mischkristallbildung können selbstverständlich in Kombination auftreten und gemeinsam zur Härtesteigerung beitragen. Durch Einstellung der jeweiligen Legierung kann dabei der Effekt der Ausscheidungshärtung den der Mischkristallbildung übersteigen und umgekehrt.

Typischerweise handelt es sich bei den erfindungsgemäß bereitgestellten Rohlingen um Scheiben, aus denen das jeweilige Implantat durch eine Fräsbearbeitung herausgearbeitet wird. Solche Scheiben werden in der Fachsprache auch als "Frässcheiben" bezeichnet.

Die Erfindung eignet sich dabei insbesondere zur Herstellung von Dentalimplantaten, die als Befestigungsmittel für Zahnersatz oder unmittelbar als Zahnersatz oder als Hilfsmittel für die Befestigung von Zahnersatz in den Kiefer eingebracht werden, oder für Dentalprothesen, die als oder für einen Zahnersatz auf dem Kiefer angebracht werden.

Co-basierte Legierungen, wie sie die Erfindung zur Verwendung als Material für Rohlinge, aus denen Implantate oder Prothesen, insbesondere Dentalimplantate oder -prothesen, durch Materialabtrag gefertigt werden, vorsieht, sind an sich aus dem Stand der Technik bekannt (z.B. EP 1 972 321 B1). Sie werden dort jedoch für andere Verwendungen oder andere Herstellverfahren eingesetzt.

Erst die Erfindung hat erkannt, dass sich diese, an sich bekannten Legierungen so zusammensetzen oder wärmebehandeln lassen, dass sie als Rohling für eine materialabtragende Bearbeitung optimierte Eigenschaften besitzen und durch eine abschließende, nach der materialabtragenden Bearbeitung folgende Wärmebehandlung auf die von den jeweiligen Implantat- oder Prothesenbauteilen geforderten mechanischen Eigenschaften gebracht werden können.

Eine für die erfindungsgemäßen Zwecke geeignete Legierungsvorschrift sieht dementsprechend vor, dass die Kobalt-Basislegierung aus (in Gew.-%)

| | |
|---|---|
| C: | bis zu 0,1 %, |
| Si: | 0,8 - 2,0 % |
| Cr: | 22 - 29 %, |
| Mn: | bis zu 1 %, |
| Ni: | bis zu 3 %, |
| Fe: | bis zu 3 %, |
| Ce: | bis zu 1 %, |
| Ga: | bis zu 5 %, |
| B: | bis zu 2,5 % |

sowie jeweils mindestens einem der härtesteigernde Ausscheidungen oder Mischkristalle bildenden Elemente aus der Gruppe "Mo, W, Nb, Al, Ti", wobei für die Gehalte dieser Elemente, sofern vorhanden, die Maßgabe gilt
Mo: 3 - 9 %,
W: 3 - 9 %,
Nb: 3 - 9 %,
AI: 0,1 - 6 %,
Ti: 0,1 - 6 %,
und als Rest Co und herstellungsbedingt unvermeidbare Verunreinigungen besteht.

Kohlenstoff kann in der erfindungsgemäß verwendeten Kobaltbasislegierung in Gehalten von bis zu 0,1 Gew.-% vorhanden sein. Kohlenstoff ist in der Regel eine unerwünschte Verunreinigung, da durch Ausscheidung von Cr-reichen Karbiden die Korrosionsbeständigkeit reduziert wird. Daher ist der C-Gehalt auf bis zu 0,1 Gew.-%, insbesondere weniger als 0,1 Gew.-% beschränkt.

Silizium ist in der erfindungsgemäß verwendeten Kobaltbasislegierung in Gehalten von 0,8 bis 2,0 Gew.-% vorhanden. Durch Zugabe von Silizium werden der Schmelzpunkt und die Viskosität der Schmelze reduziert. Gleichzeitig hat Silizium den positiven Effekt, dass die Haftung einer gegebenenfalls auf das Implantat aufgebrachten keramischen Verblendung durch Bildung von Oxiden bzw. Oxidverbindungen verbessert wird. Um diesen Effekt zu nutzen, beträgt der Si-Gehalt der Co-Legierung mindestens 0,8 Gew.-%. Oberhalb von 2 Gew.-% hat Si keine eigenschaftsverbessernden Wirkungen mehr.

Chrom ist in der erfindungsgemäß verwendeten Kobaltbasislegierung in Gehalten von 22 - 29 Gew.-%, insbesondere mehr als 22,00 Gew.-% bis weniger als 29,00 Gew.-%, enthalten. Chrom wird für die Korrosionsbeständigkeit benötigt und beteiligt sich auch an der erfindungsgemäß für die abschließende Einstellung der Härte des jeweiligen Implantats genutzten Mischkristallverfestigung. Optimale Wirkung entfaltet Cr in der erfindungsgemäß verwendeten Co-Legierung, wenn der Cr-Gehalt mindestens 23 Gew.-%, insbesondere mehr als 23,00 Gew.-%, beträgt. Gleichzeitig hat sich gezeigt, dass Cr-Gehalte von höchstens 27 Gew.-%, insbesondere weniger als 27,00 Gew.-%, eine für die Anwendung speziell im Mundraum regelmäßig ausreichende Korrosionsbeständigkeit gewährleisten. Mangan in Gehalten von bis zu 1 Gew.-% beeinflusst, ähnlich wie Silizium, Viskosität der Schmelze und die Haftung zur Verblendung. Zudem wird Schwefel durch Bildung von Mn-Sulfiden abgebunden. Die positive Wirkung von Mn in der erfindungsgemäßen Co-Legierung lässt sich insbesondere dann nutzen, wenn der Mn-Gehalt mindestens 0,01 Gew.-% beträgt. Oberhalb von 1 Gew.-% hat Mn keine eigenschaftsverbessernden Wirkungen mehr, wobei sich eine optimierte Auswirkung der Anwesenheit von Mn ergibt, wenn der Mn-Gehalt höchstens 0,5 Gew.-% beträgt.

Der Nickel-Gehalt in erfindungsgemäß verwendeter Co-Legierungen beträgt maximal 3 Gew.-%, kann aber für einen Einsatz insbesondere im Mundraum auf Gehalte von bis zu 0,1 Gew.-%, insbesondere weniger als 0,1 Gew.-%, beschränkt werden, um allergische Reaktionen sicher zu vermeiden. Co-Basislegierungen mit höheren Ni-Gehalten können aber für Knie- oder Hüftimplantate zweckmäßig sein, um die für diesen Verwendungszweck benötigten mechanischen Eigenschaften des Werkstoffs zu erreichen.

Eisen gelangt über den Herstellungsprozess in die erfindungsgemäß verwendete Co-Legierung, ist jedoch auf höchstens 3 Gew.-%, insbesondere weniger als 3,0 Gew.-%, zu beschränken, um die Korrosionsbeständigkeit des Werkstoffs nicht zu beeinträchtigen. Negative Auswirkungen der Anwesenheit von Fe können dabei dann besonders sicher vermieden werden, wenn der Fe-Gehalt auf höchstens 0,5 Gew.-%, insbesondere weniger als 0,1 Gew.-%, beschränkt wird.

Cer kann der erfindungsgemäß verwendeten Legierung in Gehalten von bis zu 1 Gew.-% zugegeben werden, da es durch Oxidbildung sehr stark die Anbindung zur keramischen Verblendung verbessert.

Gallium kann in der erfindungsgemäß verwendeten Co-Basislegierung in Gehalten von bis zu 5 Gew.-% vorhanden sein. Es beteiligt sich ebenfalls an der Mischkristallverfestigung und trägt zur Reduzierung des thermischen Ausdehnungskoeffizienten bei.

Bor hat in Gehalten von bis zu 2,5 Gew.-% mit Si vergleichbare Wirkungen. Zudem beeinflusst Bor die Oxidfarbe und bewirkt durch die Bildung von Boridausscheidungen eine Erhöhung der Härte und Festigkeit. Gehalte oberhalb von 2,5 Gew.-% sind zu vermeiden, da der Werkstoff sonst sehr spröde werden würde.

Um die erfindungsgemäß zur Einstellung der Härte des nach Durchführung eines subtraktiven Verfahrens erhaltenen Implantats genutzte Ausscheidungsbildung zu bewirken, enthält die erfindungsgemäß verwendete Co-Basislegierung mindestens ein ausscheidungsbildendes Element aus der Gruppe "Mo, W, Nb, Al, Ti", wobei diese Elemente nicht nur einzeln, sondern selbstverständlich auch in Kombination miteinander in der Co-Legierung vorhanden sein können. Beispielsweise können jeweils zwei oder mehr Elemente aus der erfindungsgemäß vorgesehenen Gruppe der ausscheidungsbildenden Elemente in der Co-Legierung enthalten sein.

Molybdän in Gehalten von 3 - 9 Gew.-% erhöht zum einen die Korrosionsbeständigkeit, bewirkt aber auch eine ausgeprägte Mischkristallverfestigung und damit einhergehend eine effektive Steigerung der Härte und Festigkeit der Co-Legierung. Gleichzeitig trägt Mo zur Reduzierung des thermischen Ausdehnungskoeffizienten bei. Besonders sicher lässt sich der positive Einfluss von Mo nutzen, wenn der Mo-Gehalt mindestens 4 Gew.-% beträgt. Praktische Versuche haben dabei belegt, dass es für die Nutzung des positiven Einfluss von Mo in der Regel ausreicht, wenn der Mo-Gehalt auf höchstens 6 Gew.-% beschränkt wird.

Wolfram in Gehalten von 3 - 9 Gew.-% hat in der erfindungsgemäß verwendeten Co-Legierung eine ähnliche Wirkung wie Molybdän und erhöht somit zum einen die Korrosionsbeständigkeit, bewirkt aber auch eine ausgeprägte Mischkristallverfestigung und damit einhergehend eine effektive Steigerung der Härte und Festigkeit der Co-Legierung. Gleichzeitig trägt W zur Reduzierung des thermischen Ausdehnungskoeffizienten bei. Besonders sicher lässt sich der positive Einfluss von W nutzen, wenn der W-Gehalt mindestens 4 Gew.-% beträgt. Praktische Versuche haben dabei belegt, dass es für die Nutzung des positiven Einfluss von W in der Regel ausreicht, wenn auch der W-Gehalt auf höchstens 6 Gew.-% beschränkt wird.

Niob kann ebenfalls in Gehalten von 3 - 9 Gew.-% in der erfindungsgemäß verwendeten Co-Legierung vorhanden sein. Zum einen führt Niob verstärkt zur Bildung von Nb-Karbiden, sofern Kohlenstoff vorhanden ist. Nicht vermeidbare Kohlenstoffverunreinigungen werden so abgebunden mit der Folge, dass die Entstehung von Cr- und/oder Mo-Karbide verhindert wird, welche die Korrosionsbeständigkeit reduzieren würden. Indirekt führt die Anwesenheit von Nb in den genannten Gehalten somit zu einer Steigerung der Korrosionsbeständigkeit. Im Übrigen entspricht die Wirkung von Nb der von Wolfram oder Molybdän. Besonders sicher lässt sich daher auch der positive Einfluss von Nb nutzen, wenn der Nb-Gehalt mindestens 4 Gew.-% beträgt. Praktische Versuche haben dabei belegt, dass es für die Nutzung des positiven Einfluss von Nb in der Regel ausreicht, wenn auch der Nb-Gehalt auf höchstens 6 Gew.-% beschränkt wird.

Aluminium und Titan beteiligen sich gleichermaßen an der Bildung von intermetallischen Phasen und unterstützt somit die Ausscheidungshärtung. Sie sind bezogen auf ihren Atom-%-Anteil im Verhältnis 1:1 austauschbar, falls erforderlich. Sofern vorhanden, beträgt der Al-Gehalt der erfindungsgemäß verwendeten Co-Legierung 0,1 - 6 Gew.-% und der Ti-Gehalt 0,1 - 6 Gew.-%, insbesondere bis zu 5 Gew.-%, wobei sich Al-Gehalte von mindestens 2 Gew.-% oder Ti-Gehalte von mindestens 1 Gew.-% als besonders wirksam erwiesen haben. Gleichzeitig hat sich gezeigt, dass Al-Gehalte von max. 4 Gew.-% oder Ti-Gehalte von max. 3 Gew.-% regelmäßig für die erfindungsgemäßen Zwecke ausreichen.

Erforderlichenfalls kann der erfindungsgemäß jeweils verwendete bzw. bereitgestellte Rohling vor der spanabhebenden Bearbeitung einer Wärmebehandlung unterzogen werden, bei der er über eine Dauer von 15 - 600 min bei einer Temperatur von 1050 - 1300 °C lösungsgeglüht wird. Optimale Lösungsglühergebnisse werden erreicht, wenn die Lösungsdauer mindestens 60 min beträgt, wobei sich eine Höchstdauer des Lösungsglühen von höchstens 480 min im Hinblick auf die Optimierung des Glühergebnisses ebenfalls bewährt hat. Zur Optimierung des Ergebnisses des Lösungsglühen trägt auch bei, wenn die Lösungsglühtemperatur mindestens 1150 °C beträgt, wobei sich unter dem Aspekt der Optimierung Lösungsglühtemperaturen von maximal 1250 °C ebenfalls bewährt haben. Das Lösungsglühen bewirkt eine Härtereduzierung durch Auflösung von intermetallischen Phasen und Kornwachstum, so dass die Rohlinge eine für die materialabhebende Bearbeitung optimale Härte aufweisen.

Die Aufheizgeschwindigkeit der Lösungsglühbehandlung sollte mindestens 5 K/min und höchstens 20 K/min betragen und damit im für konventionelle Lösungsglühöfen typischen Bereich liegen. Durch die derart erfolgende Aufheizung ist eine ausreichende Durchwärmung sichergestellt. Im Übrigen hat die Aufheizgeschwindigkeit keinen Einfluss auf die resultierenden Eigenschaften der erfindungsgemäß erzeugten Produkte. Nach dem Lösungsglühen können die Rohlinge mit einer Abkühlgeschwindigkeit von 5 - 1000 K/min auf Raumtemperatur abgekühlt werden. Wenn die Abkühlgeschwindigkeit langsamer ist, sind Ausscheidungen sowie Rekristallisation möglich. Die Folge wäre ein in diesem Arbeitsschritt unerwünschter Anstieg der Härte. Wenn deutlich schneller abgekühlt wird, könnten aufgrund der thermischen Spannungen Verzug und sogar Risse auftreten. Dabei sind die Abkühlgeschwindigkeiten innerhalb der Maßgaben der Erfindung in Abhängigkeit vom Querschnitt zu wählen und zwar jeweils so, dass auch im Kernbereich die erforderliche Abkühlung erreicht wird.

Das Lösungsglühen kann in üblicher Weise unter Normalatmosphäre durchgeführt werden. In genauso konventioneller Weise kann das Abschrecken nach dem Lösungsglühen mit Luft- oder Wasser erfolgen.

Soll jedoch der Aufwand für die Entfernung des beim konventionellen Lösungsglühen unter Normalatmosphäre entstehenden Zunders umgangen werden, so kann die Lösungsglühbehandlung unter Vakuum durchgeführt werden. Die Glühbehandlung unter Vakuum verhindert die Oxidation und Verzunderung des jeweiligen Rohlings, so dass andernfalls erforderlich werdende Nachbearbeitungen zur Zunder- oder Oxidentfernung minimiert und für die Nachbearbeitung benötigte Aufmaße vermieden werden können. Das Abschrecken kann dann mit druckbeaufschlagtem Stickstoff durchgeführt werden, um auch hierbei eine Oxidation und Zunderbildung zu vermeiden.

Die für die materialabhebende Bearbeitung optimale Härte liegt typischerweise im Bereich von bis zu 35 HRC, insbesondere bis zu 28 HRC.

Die Härte HRC wird hier in der üblichen Weise nach den in der DIN EN ISO 6508-1 bestimmten Verfahren ermittelt.

Zur Einstellung ihrer Härte können die durch materialabhebende Bearbeitung der Rohlinge erhaltenen Implantate über eine Auslagerungsdauer von mindestens 5 min, insbesondere mindestens 60 min, und höchstens 600 min bei einer 600 - 1000 °C, insbesondere bis zu 950 °C, betragenden Auslagerungstemperatur gehalten werden. Besonders bewährt haben sich dabei Auslagerungstemperaturen von mindestens 700 °C, ab denen sich die erfindungsgemäß genutzten Ausscheidungsvorgänge besonders sicher einstellen. Insbesondere kann hierzu die Auslagerungstemperatur auf mindestens 750 °C eingestellt werden.

Dabei hat sich gezeigt, dass eine Auslagerungsdauer von 5 - 150 min, insbesondere 5 - 120 min, in der Regel ausreicht, um die erfindungsgemäß genutzten Ausscheidungsvorgänge zu bewirken. Abhängig von der jeweiligen Legierungsvariante können gute Härtesteigerungen schon bei Auslagerungsdauern von höchstens 20 min erreicht werden, wobei sich bei anderen Legierungsvarianten Auslagerungsdauern von mindestens 80 min in dieser Hinsicht als vorteilhaft erwiesen haben. Die Aufheizgeschwindigkeit sollte auch hier wiederum mindestens 5 K/min und höchstens 20 K/min betragen, um mit konventionellen Öfen eine ordnungsgemäße Durchwärmung zuverlässig zu erreichen. Nach dem Auslagern können die Rohlinge mit einer Abkühlgeschwindigkeit von 5 - 20 K/min auf Raumtemperatur abgekühlt werden.

Bei Anwendungen im Bereich der Dentaltechnik sind in der Regel keine weiteren Maßnahmen erforderlich, um das für die jeweilige Verwendung angestrebte Härte- und Festigkeitsniveau zu erreichen. In anderen Fällen, beispielsweise für die Herstellung von in oder am Körper zu verbauende Befestigungsbauteile, wie Schienen, Schrauben, Spangen und desgleichen, kann es jedoch zweckmäßig sein, das jeweilige Produkt einer Kaltverfestigung durch Kaltverformung zu unterziehen, um das Härte- und Festigkeitsniveau zu steigern. In der Regel erfolgt die Kaltverfestigung vor der Auslagerung, da dann die Rohlinge bzw. die daraus geformten Produkte noch weich sind und so ein maximales Verfestigungspotenzial bieten. Als Möglichkeiten zur Kaltverformung für die Kaltverfestigung bieten sich Umformverfahren an, die bei Temperaturen von weniger als 200 °C durchgeführt werden. Hierunter fallen Verfahren, bei denen Verformungen durch Zug-, Druck- oder Biegung erzeugt werden. Zu diesen Verfahren zählen Stauchen, Ziehen, Richten, Recken und desgleichen. Typische Umformgrade liegen hier bei bis zu 80 %. Auf diese Weise sind Härtesteigerungen um bis zu 300 % möglich (beispielsweise von 20 HRC im Ausgangszustand auf 60 HRC im kaltverfestigten Zustand).

Im Fall, dass das Implantat mit einer keramischen Verblendung versehen werden soll, kann die Temperatur der Auslagerung so gewählt werden, dass die Auslagerungstemperatur der Einbrenntemperatur der jeweiligen Keramikschichten entspricht. Dann können der Vorgang der Ausscheidungshärtung oder Mischkristallbildung und des Einbrennens der Keramikverblendung miteinander kombiniert werden, so dass kein zusätzlicher Prozessschritt nötig wird und das Einbrennen der Verblendung keinen zusätzlichen Einfluss auf die Ausscheidungshärtung oder Mischkristallbildung und die dadurch eingestellten Eigenschaften des Implantats hat.

Dies ist möglich durch eine gezielt eingestellte Kombination aus chemischer Zusammensetzung, der Temperatur und der Dauer der Wärmebehandlung. Die Verblendung kann aber auch nachträglich durchgeführt werden. Die erfindungsgemäße Legierung erlaubt es hier, die Wärmebehandlung und die Prozessparameter der Verblendung mit weiten Variationsmöglichkeiten aufeinander abzustimmen. So können die optimalen Gebrauchseigenschaften eingestellt werden, ohne dass sich der Verarbeitungsprozess verkompliziert.

Um die Machbarkeit der Erfindungsmeldung aufzuzeigen wurden elf verschiedene Legierungssysteme "Variante I" - "Variante XI" gießtechnisch erzeugt. Ausgehend von einer Co-Cr-Si "Basislegierung" wurden dabei die Legierungselemente Mo, W, Nb, AI und Ti variiert.

In den Tabellen 1 - 11 ist für jede der Varianten I - XI eine allgemeine Legierungsvorschrift, die im Rahmen der allgemeinen Legierungsvorschrift als optimal angesehene Legierungsvorschrift, die Analyse der konkret untersuchten Probe sowie eine weiche und eine harte Ausgestaltung der Legierungsvorschrift angegeben. Die Einstellung der weicheren und härteren Ausgestaltungen erfolgte dabei vor allem über eine Variation der Gehalte an den Legierungselementen Al und Ti, da der Einfluss dieser Legierungselemente auf die Ausscheidungshärtung am ausgeprägtesten ist.

Die Variation von Mo, W und Nb hingegen hat vor allem einen Einfluss auf die Mischkristallverfestigung, wodurch sich die Lösungsglühhärte ändert. Mo, W und Nb bewirken vor allem in Kombination mit Al oder Ti eine ausgeprägte Ausscheidungshärtung. Ohne Al oder Ti ist eine Ausscheidungshärtung zwar auch möglich, jedoch nicht so ausgeprägt.

Bis auf die Gehalte an Ti und Al wurden die Gehalte der jeweiligen Legierungsbestandteile der jeweils konkret untersuchten Proben in an sich bekannter Weise mit einem Funkenspektrometer ermittelt. Die Gehalte an Al und Ti wurden jeweils in ebenfalls bekannter Weise nasschemisch bestimmt.

Alle Proben wurden zunächst bei 1250 °C für acht Stunden im Vakuum lösungsgeglüht. Das Aufheizen erfolgte mit einer Aufheizgeschwindigkeit von ca. 10 K/min. Das Abschrecken im Anschluss an das Lösungsglühen wurde mit 3,5 bar Stickstoff durchgeführt, was eine Abkühlgeschwindigkeit 30 - 50 K/min bei den hier durchgeführten Versuchen entsprach.

In der Praxis würde sich nach dem Lösungsglühen die materialabhebende Bearbeitung des Rohlings zur Herstellung des Implantats anschließen.

Die anschließenden Auslagerungsversuche wurden im Temperaturbereich von 500 - 1000 °C (in 100 °C-Schritten) für jeweils zehn Stunden durchgeführt. Die Aufheiz- und Abkühlgeschwindigkeiten betrugen dabei jeweils ca. 10 K/min. Nach jeder Wärmebehandlung wurde die Härte in HRC gemessen. In den Tabellen 1 - 11 sind zu den konkret untersuchten Analysen jeweils die Mittelwerte aus mindestens fünf Einzelmessungen angegeben. Variante V ist dabei nicht erfindungsgemäß, da sie kein Element aus der erfindungsgemäß vorgesehenen Gruppe der ausscheidungshärtenden oder eine Verfestigung durch Mischkristallbildung bewirkenden Elemente enthält.

Die in den Tabelle 1-11 dargestellten Ergebnisse zeigen, dass vor allem die AI- und Ti- haltigen Legierungen zu einer ausgeprägten Ausscheidungshärtung neigen, wenn die Auslagerung im Temperaturbereich von etwa 800 °C durchgeführt wird.

Die Varianten III und V zeigen zwar nur einen vergleichbar geringen Anstieg der Härte nach der Auslagerungsbehandlung, sind aber insbesondere für die Härtesteigerung durch Kaltverfestigung geeignet. So konnte gezeigt werden, dass bei den Varianten III und V durch Kaltverfestigung der Proben eine Härtesteigerung auf bis zu 60 HRC erzielt werden kann.

Aufbauend auf den konkret untersuchten Proben der Varianten VII - XI wurden weitere Auslagerungsversuche durchgeführt, um die Härtesteigerung in Abhängigkeit der Auslagerungsdauer zu untersuchen. Letztere wurde in 20 min-Schritten bis insgesamt 120 Minuten variiert. Die Versuche gingen ebenfalls von dem Zustand aus, der nach dem schon erläuterten Lösungsglühen bei 1250 °C erreicht worden ist. Die übrigen Parameter der Wärmebehandlung wurden ebenfalls nicht verändert.

Die Ergebnisse der Auslagerungsversuche sind in Tabelle 12 dargestellt. Es ist zu erkennen, dass bereits Auslagerungszeiten von mehr als 60 Minuten eine deutliche Härtesteigerung bewirken. Bei den Varianten VII - X liegt nach einer Auslagerung von 120 Minuten bei 800 °C schon die gleiche Härte vor, wie bei einer Auslagerung nach zehn Stunden.

Die Erfindung zeigt somit Möglichkeiten auf, die eine vereinfachte Herstellung von Implantaten oder Prothesen, speziell von Dentalimplantaten oder -prothesen, durch Anwendung subtraktiver Verfahren erlauben. Hierzu schlägt die Erfindung die Verwendung einer ausscheidungshärtenden oder durch Mischkristallbildung verfestigenden, biokompatiblen Kobalt-Basislegierung für die Herstellung von Rohlingen vor. Solche Rohlinge werden im Zuge des erfindungsgemäßen Verfahrens im weichen Zustand bereitgestellt. Anschließend wird aus dem weichen Rohling das jeweilige Bauteil (Implantat oder Prothese) durch materialabhebende Bearbeitung hergestellt. Abschließend erfolgt dann eine Wärmebehandlung des Implantats oder der Prothese zur Einstellung seiner Härte durch Ausscheidungshärtung oder Mischkristallbildung.

**Tabelle 1**

| **VARIANTE I** | C | Si | Cr | Mo | W | Nb | Al | Ti | Mn | Ni | Fe |
|---|---|---|---|---|---|---|---|---|---|---|---|
| Allgemein *) | <0,5 | ≤2,5 | 22 - 29 | 3,0 - 9,0 | 3,0 - 9,0 | - | - | - | <1,0 | <3,0 | <3,0 |
| Optimal *) | <0,1 | 0,8-2,0 | 23 - 27 | 4,0 - 6,0 | 4,0 - 6,0 | - | - | - | <0,5 | <0,1 | <0,5 |
| Weiche Variante *) | <0,1 | 0,8-2,0 | 23 - 27 | 3,0 - 5,0 | 3,0 - 5,0 | - | - | - | <0,5 | <0,1 | <0,5 |
| Harte Variante *) | <0,1 | 0,8-2,0 | 23 - 27 | 5,0 - 7,0 | 5,0 - 7,0 | - | - | - | <0,5 | <0,1 | <0,5 |
| untersuchten Probe *) | 0,032 | 0,99 | 24,61 | 5,08 | 4,98 | - | - | - | <0,01 | <0,01 | 0,06 |

| Lösungsglühung | Dauer | Temperatur | | Härte HRC nach Lösungsglühung | | | | | | | |
|---|---|---|---|---|---|---|---|---|---|---|---|
| | 8 | 1250 | | 18,3 ± 2,0 | | | | | | | |

| Auslagerung | Dauer | Temperatur | | Härte HRC nach Auslagerung | | | | | | | |
|---|---|---|---|---|---|---|---|---|---|---|---|
| Versuch 1 | 10h | 500 °C | | 19,5 ± 2,7 | | | | | | | |
| Versuch 2 | 10h | 600 °C | | 19,4 ± 2,6 | | | | | | | |
| Versuch 3 | 10h | 700 °C | | 22,7 ± 2,2 | | | | | | | |
| Versuch 4 | 10h | 800 °C | | 31,2 ± 3,1 | | | | | | | |
| Versuch 5 | 10h | 900 °C | | 28,5 ± 1,8 | | | | | | | |
| Versuch 6 | 10h | 1000 °C | | 21,8 ± 1,0 | | | | | | | |

| | | | | | | | | | | | |
|---|---|---|---|---|---|---|---|---|---|---|---|
| *) Angaben in Gew.-%, Rest Co und unvermeidbare Verunreinigungen | | | | | | | | | | | |

**Tabelle 2**

| **VARIANTE II** | C | Si | Cr | Mo | W | Nb | Al | Ti | Mn | Ni | Fe |
|---|---|---|---|---|---|---|---|---|---|---|---|
| Allgemein *) | <0,5 | ≤2,5 | 22 - 29 | 3,0 - 9,0 | - | - | - | - | <1,0 | <3,0 | <3,0 |
| Optimal *) | <0,1 | 0,8-2,0 | 23 - 27 | 4,0 - 6,0 | - | - | - | - | <0,5 | <0,1 | <0,5 |
| Weiche Variante *) | <0,1 | 0,8-2,0 | 23 - 27 | 3,0 - 5,0 | - | - | - | - | <0,5 | <0,1 | <0,5 |
| Harte Variante *) | <0,1 | 0,8-2,0 | 23 - 27 | 5,0 - 7,0 | - | - | - | - | <0,5 | <0,1 | <0,5 |
| untersuchten Probe *) | 0,005 | 1,01 | 24,01 | 5,02 | - | - | - | - | <0,01 | <0,01 | 0,06 |

| Lösungsglühung | Dauer | Temperatur | | Härte HRC nach Lösungsglühung | | | | | | | |
|---|---|---|---|---|---|---|---|---|---|---|---|
| | 8 | 1250 | | 21,9 ± 2,3 | | | | | | | |

| Auslagerung | Dauer | Temperatur | | Härte HRC nach Auslagerung | | | | | | | |
|---|---|---|---|---|---|---|---|---|---|---|---|
| Versuch 1 | 10h | 500 °C | | 24,3 ± 1,5 | | | | | | | |
| Versuch 2 | 10h | 600 °C | | 25,3 ± 1,5 | | | | | | | |
| Versuch 3 | 10h | 700 °C | | 22,6 ± 1,8 | | | | | | | |
| Versuch 4 | 10h | 800 °C | | 22,7 ± 1,6 | | | | | | | |
| Versuch 5 | 10h | 900 °C | | 25,8 ± 1,8 | | | | | | | |
| Versuch 6 | 10h | 1000 °C | | 21,3 ± 1,7 | | | | | | | |

| | | | | | | | | | | | |
|---|---|---|---|---|---|---|---|---|---|---|---|
| *) Angaben in Gew.-%, Rest Co und unvermeidbare Verunreinigungen | | | | | | | | | | | |

**Tabelle 3**

| **VARIANTE III** | C | Si | Cr | Mo | W | Nb | Al | Ti | Mn | Ni | Fe |
|---|---|---|---|---|---|---|---|---|---|---|---|
| Allgemein *) | <0,5 | ≤2,5 | 22 - 29 | - | 3,0 - 9,0 | - | - | - | <1,0 | <3,0 | <3,0 |
| Optimal *) | <0,1 | 0,8-2,0 | 23 - 27 | - | 4,0 - 6,0 | - | - | - | <0,5 | <0,1 | <0,5 |
| Weiche Variante *) | <0,1 | 0,8-2,0 | 23 - 27 | - | 3,0 - 5,0 | - | - | - | <0,5 | <0,1 | <0,5 |
| Harte Variante *) | <0,1 | 0,8-2,0 | 23 - 27 | - | 5,0 - 7,0 | - | - | - | <0,5 | <0,1 | <0,5 |
| untersuchten Probe *) | 0,011 | 0,96 | 24,89 | - | 5,16 | - | - | - | <0,01 | <0,02 | 0,06 |

| Lösungsglühung | Dauer | Temperatur | | Härte HRC nach Lösungsglühung | | | | | | | |
|---|---|---|---|---|---|---|---|---|---|---|---|
| | 8 | 1250 | | 22,1 ± 2,1 | | | | | | | |

| Auslagerung | Dauer | Temperatur | | Härte HRC nach Auslagerung | | | | | | | |
|---|---|---|---|---|---|---|---|---|---|---|---|
| Versuch 1 | 10h | 500 °C | | 22,8 ± 0,5 | | | | | | | |
| Versuch 2 | 10h | 600 °C | | 23,3 ± 2,5 | | | | | | | |
| Versuch 3 | 10h | 700 °C | | 23,3 ± 3,2 | | | | | | | |
| Versuch 4 | 10h | 800 °C | | 20,7 ± 2,7 | | | | | | | |
| Versuch 5 | 10h | 900 °C | | 21,7 ± 1,7 | | | | | | | |
| Versuch 6 | 10h | 1000 °C | | 22,9 ± 3,1 | | | | | | | |

| | | | | | | | | | | | |
|---|---|---|---|---|---|---|---|---|---|---|---|
| *) Angaben in Gew.-%, Rest Co und unvermeidbare Verunreinigungen | | | | | | | | | | | |

**Tabelle 4**

| **VARIANTE IV** | C | Si | Cr | Mo | W | Nb | Al | Ti | Mn | Ni | Fe |
|---|---|---|---|---|---|---|---|---|---|---|---|
| Allgemein *) | <0,5 | ≤2,5 | 22 - 29 | - | - | 3,0 - 9,0 | - | - | <1,0 | <3,0 | <3,0 |
| Optimal *) | <0,1 | 0,8-2,0 | 23 - 27 | - | - | 4,0 - 6,0 | - | - | <0,5 | <0,1 | <0,5 |
| Weiche Variante *) | <0,1 | 0,8-2,0 | 23 - 27 | - | - | 3,0 - 5,0 | - | - | <0,5 | <0,1 | <0,5 |
| Harte Variante *) | <0,1 | 0,8-2,0 | 23 - 27 | - | - | 5,0 - 7,0 | - | - | <0,5 | <0,1 | <0,5 |
| untersuchten Probe *) | 0,005 | 1,02 | 25,27 | - | - | 5,46 | - | - | <0,01 | <0,02 | 0,07 |

| Lösungsglühung | Dauer | Temperatur | | Härte HRC nach Lösungsglühung | | | | | | | |
|---|---|---|---|---|---|---|---|---|---|---|---|
| | 8 | 1250 | | 32,1 ± 0,9 | | | | | | | |

| Auslagerung | Dauer | Temperatur | | Härte HRC nach Auslagerung | | | | | | | |
|---|---|---|---|---|---|---|---|---|---|---|---|
| Versuch 1 | 10h | 500 °C | | 30,0 ± 1,1 | | | | | | | |
| Versuch 2 | 10h | 600 °C | | 30,3 ± 1,3 | | | | | | | |
| Versuch 3 | 10h | 700 °C | | 43,3 ± 1,5 | | | | | | | |
| Versuch 4 | 10h | 800 °C | | 46,0 ± 1,0 | | | | | | | |
| Versuch 5 | 10h | 900 °C | | 42,1 ± 1,8 | | | | | | | |
| Versuch 6 | 10h | 1000 °C | | 36,1 ± 1,6 | | | | | | | |

| | | | | | | | | | | | |
|---|---|---|---|---|---|---|---|---|---|---|---|
| *) Angaben in Gew.-%, Rest Co und unvermeidbare Verunreinigungen; | | | | | | | | | | | |

**Tabelle 5**

| **VARIANTE V nicht erfindungsgemäß** | C | Si | Cr | Mo | W | Nb | Al | Ti | Mn | Ni | Fe |
|---|---|---|---|---|---|---|---|---|---|---|---|
| Allgemein *) | <0,5 | ≤2,5 | 22 - 29 | - | - | - | - | - | <1,0 | <3,0 | <3,0 |
| Optimal *) | <0,1 | 0,8-2,0 | 23 - 27 | - | - | - | - | - | <0,5 | <0,1 | <0,5 |
| Weiche Variante *) | <0,1 | 0,8-2,0 | 23 - 27 | - | - | - | - | - | <0,5 | <0,1 | <0,5 |
| Harte Variante *) | <0,1 | 0,8-2,0 | 23 - 27 | - | - | - | - | - | <0,5 | <0,1 | <0,5 |
| untersuchten Probe *) | 0,011 | 0,96 | 24,89 | - | - | - | - | - | <0,01 | <0,02 | 0,07 |

| Lösungsglühung | Dauer | Temperatur | | Härte HRC nach Lösungsglühung | | | | | | | |
|---|---|---|---|---|---|---|---|---|---|---|---|
| | 8 | 1250 | | 18,6 ± 1,7 | | | | | | | |

| Auslagerung | Dauer | Temperatur | | Härte HRC nach Auslagerung | | | | | | | |
|---|---|---|---|---|---|---|---|---|---|---|---|
| Versuch 1 | 10h | 500 °C | | 215 ± 1,7 | | | | | | | |
| Versuch 2 | 10h | 600 °C | | 20,6 ± 2,7 | | | | | | | |
| Versuch 3 | 10h | 700 °C | | 20,0 ± 1,4 | | | | | | | |
| Versuch 4 | 10h | 800 °C | | 19,8 ± 1,7 | | | | | | | |
| Versuch 5 | 10h | 900 °C | | 22,7 ± 2,3 | | | | | | | |
| Versuch 6 | 10h | 1000 °C | | 22,5 ± 2,4 | | | | | | | |

| | | | | | | | | | | | |
|---|---|---|---|---|---|---|---|---|---|---|---|
| *) Angaben in Gew.-%, Rest Co und unvermeidbare Verunreinigungen | | | | | | | | | | | |

**Tabelle 6**

| **VARIANTE VI** | C | Si | Cr | Mo | W | Nb | Al | Ti | Mn | Ni | Fe |
|---|---|---|---|---|---|---|---|---|---|---|---|
| Allgemein *) | <0,5 | ≤2,5 | 22 - 29 | - | - | | 0,1 -6,0 | - | <1,0 | <3,0 | <3,0 |
| Optimal *) | <0,1 | 0,8-2,0 | 23 - 27 | - | - | | 2,0 - 4,0 | - | <0,5 | <0,1 | <0,5 |
| Weiche Variante *) | <0,1 | 0,8-2,0 | 23 - 27 | - | - | | 0,1 - 3,0 | - | <0,5 | <0,1 | <0,5 |
| Harte Variante *) | <0,1 | 0,8-2,0 | 23 - 27 | - | - | | 3,0 - 6,0 | - | <0,5 | <0,1 | <0,5 |
| untersuchten Probe *) | 0,005 | 1,02 | 25,27 | - | - | | 3,58 | - | <0,01 | <0,02 | 0,07 |

| Lösungsglühung | Dauer | Temperatur | | Härte HRC nach Lösungsglühung | | | | | | | |
|---|---|---|---|---|---|---|---|---|---|---|---|
| | 8 | 1250 | | 19,3 ± 2,0 | | | | | | | |

| Auslagerung | Dauer | Temperatur | | Härte HRC nach Auslagerung | | | | | | | |
|---|---|---|---|---|---|---|---|---|---|---|---|
| Versuch 1 | 10h | 500 °C | | 19,0 ± 1,4 | | | | | | | |
| Versuch 2 | 10h | 600 °C | | 19,0 ± 1,8 | | | | | | | |
| Versuch 3 | 10h | 700 °C | | 20,7 ± 2,2 | | | | | | | |
| Versuch 4 | 10h | 800 °C | | 25,1 ± 2,6 | | | | | | | |
| Versuch 5 | 10h | 900 °C | | 30,1 ± 2,3 | | | | | | | |
| Versuch 6 | 10h | 1000 °C | | 24,4 ± 1,1 | | | | | | | |

| | | | | | | | | | | | |
|---|---|---|---|---|---|---|---|---|---|---|---|
| *) Angaben in Gew.-%, Rest Co und unvermeidbare Verunreinigungen | | | | | | | | | | | |

**Tabelle 7**

| **VARIANTE VII** | C | Si | Cr | Mo | W | Nb | Al | Ti | Mn | Ni | Fe |
|---|---|---|---|---|---|---|---|---|---|---|---|
| Allgemein *) | <0,5 | ≤2,5 | 22 - 29 | 3,0 - 9,0 | - | - | 0,1 - 6,0 | - | <1,0 | <3,0 | <3,0 |
| Optimal *) | <0,1 | 0,8-2,0 | 23 - 27 | 4,0 - 6,0 | - | - | 2,0 - 4,0 | - | <0,5 | <0,1 | <0,5 |
| Weiche Variante *) | <0,1 | 0,8-2,0 | 23 - 27 | 4,0 - 6,0 | - | - | 0,1 - 3,0 | - | <0,5 | <0,1 | <0,5 |
| Harte Variante *) | <0,1 | 0,8-2,0 | 23 - 27 | 4,0 - 6,0 | - | - | 3,0 - 6,0 | - | <0,5 | <0,1 | <0,5 |
| untersuchten Probe *) | 0,019 | 1,23 | 25,06 | 4,76 | - | - | 3,62 | - | <0,01 | <0,01 | 0,11 |

| Lösungsglühung | Dauer | Temperatur | | Härte HRC nach Lösungsglühung | | | | | | | |
|---|---|---|---|---|---|---|---|---|---|---|---|
| | 8 | 1250 | | 20,4 ± 1,5 | | | | | | | |

| Auslagerung | Dauer | Temperatur | | Härte HRC nach Auslagerung | | | | | | | |
|---|---|---|---|---|---|---|---|---|---|---|---|
| Versuch 1 | 10h | 500 °C | | 17,3 ± 2,3 | | | | | | | |
| Versuch 2 | 10h | 600 °C | | 17,5 ± 2,7 | | | | | | | |
| Versuch 3 | 10h | 700 °C | | 20,3 ± 1,4 | | | | | | | |
| Versuch 4 | 10h | 800 °C | | 41,8 ± 2,6 | | | | | | | |
| Versuch 5 | 10h | 900 °C | | 43,4 ± 1,7 | | | | | | | |
| Versuch 6 | 10h | 1000 °C | | 35,7 ± 1,1 | | | | | | | |

| | | | | | | | | | | | |
|---|---|---|---|---|---|---|---|---|---|---|---|
| *) Angaben in Gew.-%, Rest Co und unvermeidbare Verunreinigungen | | | | | | | | | | | |

**Tabelle 8**

| **VARIANTE VIII** | C | Si | Cr | Mo | W | Nb | Al | Ti | Mn | Ni | Fe |
|---|---|---|---|---|---|---|---|---|---|---|---|
| Allgemein *) | <0,5 | ≤2,5 | 22 - 29 | - | 3,0 - 9,0 | - | 0,1 -6,0 | - | <1,0 | <3,0 | <3,0 |
| Optimal *) | <0,1 | 0,8-2,0 | 23 - 27 | - | 4,0 - 6,0 | - | 2,0 - 4,0 | - | <0,5 | <0,1 | <0,5 |
| Weiche Variante *) | <0,1 | 0,8-2,0 | 23 - 27 | - | 4,0 - 6,0 | - | 0,1 - 3,0 | - | <0,5 | <0,1 | <0,5 |
| Harte Variante *) | <0,1 | 0,8-2,0 | 23 - 27 | - | 4,0 - 6,0 | - | 3,0 - 6,0 | - | <0,5 | <0,1 | <0,5 |
| untersuchten Probe *) | 0,036 | 1,26 | 25,29 | - | 4,92 | - | 3,4 | - | <0,01 | 0,03 | 0,07 |

| Lösungsglühung | Dauer | Temperatur | | Härte HRC nach Lösungsglühung | | | | | | | |
|---|---|---|---|---|---|---|---|---|---|---|---|
| | 8 | 1250 | | 21,6 ± 1,2 | | | | | | | |

| Auslagerung | Dauer | Temperatur | | Härte HRC nach Auslagerung | | | | | | | |
|---|---|---|---|---|---|---|---|---|---|---|---|
| Versuch 1 | 10h | 500 °C | | 18,7 ± 1,4 | | | | | | | |
| Versuch 2 | 10h | 600 °C | | 17,3 ± 1,2 | | | | | | | |
| Versuch 3 | 10h | 700 °C | | 18,4 ± 2,1 | | | | | | | |
| Versuch 4 | 10h | 800 °C | | 29,6 ± 2,1 | | | | | | | |
| Versuch 5 | 10h | 900 °C | | 36,1 ± 1,3 | | | | | | | |
| Versuch 6 | 10h | 1000 °C | | 31,4 ± 1,5 | | | | | | | |

| | | | | | | | | | | | |
|---|---|---|---|---|---|---|---|---|---|---|---|
| *) Angaben in Gew.-%, Rest Co und unvermeidbare Verunreinigungen | | | | | | | | | | | |

**Tabelle 9**

| **VARIANTE IX** | C | Si | Cr | Mo | W | Nb | Al | Ti | Mn | Ni | Fe |
|---|---|---|---|---|---|---|---|---|---|---|---|
| Allgemein *) | <0,5 | ≤2,5 | 22 - 29 | - | - | 3,0 - 9,0 | 0,1 - 6,0 | - | <1,0 | <3,0 | <3,0 |
| Optimal *) | <0,1 | 0,8-2,0 | 23 - 27 | - | - | 4,0 - 6,0 | 2,0 - 4,0 | - | <0,5 | <0,1 | <0,5 |
| Weiche Variante *) | <0,1 | 0,8-2,0 | 23 - 27 | - | - | 4,0 - 6,0 | 0,1 - 3,0 | - | <0,5 | <0,1 | <0,5 |
| Harte Variante *) | <0,1 | 0,8-2,0 | 23 - 27 | - | - | 4,0 - 6,0 | 3,0 - 6,0 | - | <0,5 | <0,1 | <0,5 |
| untersuchten Probe *) | 0,019 | 1,40 | 25,39 | - | - | 5,73 | 3,50 | - | <0,01 | 0,04 | 0,08 |

| Lösungsglühung | Dauer | Temperatur | | Härte HRC nach Lösungsglühung | | | | | | | |
|---|---|---|---|---|---|---|---|---|---|---|---|
| | 8 | 1250 | | 27,0 ± 1,5 | | | | | | | |

| Auslagerung | Dauer | Temperatur | | Härte HRC nach Auslagerung | | | | | | | |
|---|---|---|---|---|---|---|---|---|---|---|---|
| Versuch 1 | 10h | 500 °C | | 26,6 ± 2,6 | | | | | | | |
| Versuch 2 | 10h | 600 °C | | 29,3 ± 3,1 | | | | | | | |
| Versuch 3 | 10h | 700 °C | | 29,0 ± 2,8 | | | | | | | |
| Versuch 4 | 10h | 800 °C | | 46,1 ± 1,8 | | | | | | | |
| Versuch 5 | 10h | 900 °C | | 43,5 ± 1,6 | | | | | | | |
| Versuch 6 | 10h | 1000 °C | | 39,3 ± 1,8 | | | | | | | |

| | | | | | | | | | | | |
|---|---|---|---|---|---|---|---|---|---|---|---|
| *) Angaben in Gew.-%, Rest Co und unvermeidbare Verunreinigungen | | | | | | | | | | | |

**Tabelle 10**

| **VARIANTE X** | C | Si | Cr | Mo | W | Nb | Al | Ti | Mn | Ni | Fe |
|---|---|---|---|---|---|---|---|---|---|---|---|
| Allgemein *) | <0,5 | ≤2,5 | 22-29 | 3,0 - 9,0 | 3,0 - 9,0 | - | 0,1 - 6,0 | - | <1,0 | <3,0 | <3,0 |
| Optimal *) | <0,1 | 0,8-2,0 | 23 - 27 | 4,0 - 6,0 | 4,0 - 6,0 | - | 2,0 - 4,0 | - | <0,5 | <0,1 | <0,5 |
| Weiche Variante *) | <0,1 | 0,8-2,0 | 23 - 27 | 4,0 - 6,0 | 4,0 - 6,0 | - | 0,1 -3,0 | - | <0,5 | <0,1 | <0,5 |
| Harte Variante *) | <0,1 | 0,8-2,0 | 23 - 27 | 4,0 - 6,0 | 4,0 - 6,0 | - | 3,0 - 6,0 | - | <0,5 | <0,1 | <0,5 |
| untersuchten Probe *) | 0,045 | 1,35 | 25,02 | 4,85 | 4,83 | - | 3,46 | - | <0,01 | 0,05 | 0,07 |

| Lösungsglühung | Dauer | Temperatur | | Härte HRC nach Lösungsglühung | | | | | | | |
|---|---|---|---|---|---|---|---|---|---|---|---|
| | 8 | 1250 | | 34,0 ± 1,2 | | | | | | | |

| Auslagerung | Dauer | Temperatur | | Härte HRC nach Auslagerung | | | | | | | |
|---|---|---|---|---|---|---|---|---|---|---|---|
| Versuch 1 | 10h | 500 °C | | 30,7 ± 2,7 | | | | | | | |
| Versuch 2 | 10h | 600 °C | | 33,0 ± 1,4 | | | | | | | |
| Versuch 3 | 10h | 700 °C | | 34,9 ± 1,5 | | | | | | | |
| Versuch 4 | 10h | 800 °C | | 53,7 ± 1,3 | | | | | | | |
| Versuch 5 | 10h | 900 °C | | 51,7 ± 2,1 | | | | | | | |
| Versuch 6 | 10h | 1000 °C | | 47,1 ± 1,7 | | | | | | | |

| | | | | | | | | | | | |
|---|---|---|---|---|---|---|---|---|---|---|---|
| *) Angaben in Gew.-%, Rest Co und unvermeidbare Verunreinigungen | | | | | | | | | | | |

**Tabelle 11**

| **VARIANTE XI** | C | Si | Cr | Mo | W | Nb | Al | Ti | Mn | Ni | Fe |
|---|---|---|---|---|---|---|---|---|---|---|---|
| Allgemein *) | <0,5 | ≤2,5 | 22 - 29 | 3,0 - 9,0 | - | - | - | 0,1 - 5,0 | <1,0 | <3,0 | <3,0 |
| Optimal *) | <0,1 | 0,8-2,0 | 23 - 27 | 4,0 - 6,0 | - | - | - | 1,0 - 3,0 | <0,5 | <0,1 | <0,5 |
| Weiche Variante *) | <0,1 | 0,8-2,0 | 23 - 27 | 4,0 - 6,0 | - | - | - | 0,1 - 2,0 | <0,5 | <0,1 | <0,5 |
| Harte Variante *) | <0,1 | 0,8-2,0 | 23 - 27 | 4,0 - 6,0 | - | - | - | 2,0 - 5,0 | <0,5 | <0,1 | <0,5 |
| untersuchten Probe *) | 0,005 | 1,97 | 25,02 | 5,07 | - | - | - | 1,86 | <0,01 | 0,03 | 0,08 |

| Lösungsglühung | Dauer | Temperatur | | Härte HRC nach Lösungsglühung | | | | | | | |
|---|---|---|---|---|---|---|---|---|---|---|---|
| | 8 | 1250 | | 25,2 ± 0,4 | | | | | | | |

| Auslagerung | Dauer | Temperatur | | Härte HRC nach Auslagerung | | | | | | | |
|---|---|---|---|---|---|---|---|---|---|---|---|
| Versuch 1 | 10h | 500 °C | | 22,0 ± 1,9 | | | | | | | |
| Versuch 2 | 10h | 600 °C | | 27,0 ± 1,8 | | | | | | | |
| Versuch 3 | 10h | 700 °C | | 30,9 ± 1,8 | | | | | | | |
| Versuch 4 | 10h | 800 °C | | 48,0 ± 2,5 | | | | | | | |
| Versuch 5 | 10h | 900 °C | | 44,2 ± 2,0 | | | | | | | |
| Versuch 6 | 10h | 1000 °C | | 40,8 ± 2,9 | | | | | | | |

| | | | | | | | | | | | |
|---|---|---|---|---|---|---|---|---|---|---|---|
| *) Angaben in Gew.-%, Rest Co und unvermeidbare Verunreinigungen | | | | | | | | | | | |

**Tabelle 12**

| Variante: | VII | VIII | IX | X | XI |
|---|---|---|---|---|---|
| Härte HRC nach Lösungsglühung: | 20,4 ± 1,5 | 21,6 ± 1,2 | 27,0 ± 1,5 | 34,0 ± 1,2 | 25,2 ± 0,4 |

| Auslagerung Temperatur / Dauer | Härte HRC nach Auslagerung: | | | | |
|---|---|---|---|---|---|
| 800 °C / 20 min | 20,2 ± 1,2 | 18,3 ± 0,5 | 27,5 ± 2,9 | 32,8 ± 1,3 | 21,3 ± 1,4 |
| 800 °C / 40 min | 21,0 ± 1,9 | 19,0 ± 2,0 | 30,6 ± 4,0 | 33,2 ± 0,8 | 23,8 ± 1,8 |
| 800 °C / 60 min | 21,0 ± 2,2 | 17,8 ± 1,0 | 32,8 ± 2,5 | 35,3 ± 2,1 | 24,0 ± 2,4 |
| 800 °C / 80 min | 31,7 ± 3,4 | 23,4 ± 2,3 | 40,8 ± 2,8 | 50,3 ± 2,2 | 35,0 ± 1,6 |
| 800 °C / 100 min | 41,3 ± 1,5 | 29,3 ± 2,2 | 43,7 ± 0,8 | 50,3 ± 2,2 | 35,0 ± 1,6 |
| 800 °C / 120 min | 42,8 ± 1,9 | 29,0 ± 1,7 | 46,0 ± 1,1 | 52,8 ± 2,0 | 36,4 ± 1,5 |
| 800 °C / 10 h | 41,8 ± 2,6 | 29,6 ± 2,1 | 46,1 ± 1,8 | 53,7 ± 1,3 | 48,0 ± 2,5 |

## Patentansprüche

1. Verwendung einer ausscheidungshärtenden oder durch Mischkristallbildung verfestigenden, biokompatiblen Kobalt-Basislegierung für die Herstellung von Rohlingen, die dazu vorgesehen sind, dass aus ihnen im weichen Zustand durch materialabhebende Bearbeitung Implantate oder Prothesen herauszuarbeitet werden, welche nach der materialabhebenden Bearbeitung einer Wärmebehandlung unterzogen werden, um die Endhärte der Implantate oder Prothesen durch Ausscheidungshärtung oder Mischkristallbildung einzustellen, wobei die Kobalt-Basislegierung aus (in Gew.-%)
| | |
|---|---|
| C: | bis zu 0,1 %, |
| Si: | 0,8 - 2,0 % |
| Cr: | 22 - 29 %, |
| Mn: | bis zu 1 %, |
| Ni: | bis zu 3 %, |
| Fe: | bis zu 3 %, |
| Ce: | bis zu 1 %, |
| Ga: | bis zu 5 %, |
| B: | bis zu 2,5 % |
sowie jeweils mindestens einem der härtesteigernde Ausscheidungen oder Mischkristalle bildenden Elemente aus der Gruppe "Mo, W, Nb, AI, Ti", wobei für die Gehalte dieser Elemente, sofern vorhanden, die Maßgabe gilt
Mo:3 - 9 %,
W: 3 - 9 %,
Nb: 3 - 9 %,
Al: 0,1 - 6 %,
Ti: 0,1 - 6 %,
und als Rest Co und herstellungsbedingt unvermeidbare Verunreinigungen besteht.

2. Verfahren zum Herstellen eines Implantats oder einer Prothese, umfassend folgende Arbeitsschritte:
- Bereitstellen eines weichen Rohlings, der aus einer ausscheidungshärtenden oder durch Mischkristallbildung verfestigenden, biokompatiblen Legierung auf Basis von Kobalt besteht,
- Herausarbeiten eines Implantats oder einer Prothese aus dem weichen Rohling durch materialabhebende Bearbeitung,
- Wärmebehandeln des Implantats oder der Prothese zur Einstellung der Härte durch Ausscheidungshärtung oder Mischkristallbildung,
wobei die Kobalt-Basislegierung aus (in Gew.-%)
| | |
|---|---|
| C: | bis zu 0,1 %, |
| Si: | 0,8 - 2,0 % |
| Cr: | 22 - 29 %, |
| Mn: | bis zu 1 %, |
| Ni: | bis zu 3 %, |
| Fe: | bis zu 3 %, |
| Ce: | bis zu 1 %, |
| Ga: | bis zu 5 %, |
| B: | bis zu 2,5 % |
sowie jeweils mindestens einem der härtesteigernde Ausscheidungen oder Mischkristalle bildenden Elemente aus der Gruppe "Mo, W, Nb, AI, Ti", wobei für die Gehalte dieser Elemente, sofern vorhanden, die Maßgabe gilt
Mo:3 - 9 %,
W: 3 - 9 %,
Nb: 3 - 9 %,
Al: 0,1 - 6 %,
Ti: 0,1 - 6 %,
und als Rest Co und herstellungsbedingt unvermeidbare Verunreinigungen besteht.

3. Verwendung oder Verfahren nach Anspruch 1 oder 2, **dadurch gekennzeichnet, dass** die Rohlinge Scheiben sind, aus denen das jeweilige Implantat oder die jeweilige Prothese durch eine Fräsbearbeitung herausgearbeitet wird.

4. Verwendung oder Verfahren nach einem der voranstehenden Ansprüche, **dadurch gekennzeichnet, dass** der Cr-Gehalt der Kobalt-Basislegierung 23 - 27 Gew.-% beträgt.

5. Verwendung oder Verfahren nach einem der voranstehenden Ansprüche, **dadurch gekennzeichnet, dass** für die Gehalte des oder der jeweils in der Kobalt-Basislegierung vorhandenen härtesteigernde Ausscheidungen oder Mischkristalle bildenden Elemente folgende Maßgabe gilt (in Gew.-%)
Mo:4 - 6 %,
W: 4 - 6 %,
Nb: 4 - 6 %,
AI: 2 - 4 %,
Ti: 1 - 3 %.

6. Verwendung oder Verfahren nach einem der voranstehenden Ansprüche, **dadurch gekennzeichnet, dass** (in Gew.-%) der Mn-Gehalt der Kobalt-Basislegierung höchstens 0,5 % oder der Ni-Gehalt der Kobalt-Basislegierung höchstens 0,1 % beträgt.

7. Verwendung oder Verfahren nach einem der voranstehenden Ansprüche, **dadurch gekennzeichnet, dass** jeweils mindestens zwei härtesteigernde Ausscheidungen bildende Elemente in der Kobalt-Basislegierung vorhanden sind.

8. Verwendung oder Verfahren nach einem der voranstehenden Ansprüche, **dadurch gekennzeichnet, dass** die Rohlinge vor ihrer materialabhebenden Bearbeitung bei einer Temperatur von 1050 - 1300°C über eine Dauer von 15 - 600 min lösungsgeglüht werden.

9. Verwendung oder Verfahren nach Anspruch 8, **dadurch gekennzeichnet, dass** die Rohlinge nach dem Lösungsglühen mit einer Abkühlgeschwindigkeit von 5 - 1000 K/min auf Raumtemperatur abgekühlt werden.

10. Verwendung oder Verfahren nach einem der voranstehenden Ansprüche, **dadurch gekennzeichnet, dass** die Implantate oder die Prothesen zur Einstellung ihrer Härte über eine Auslagerungsdauer von mindestens 5 min und höchstens 600 min bei einer 600 - 1000 °C betragenden Auslagerungstemperatur gehalten werden.

11. Verwendung oder Verfahren nach Anspruch 10, **dadurch gekennzeichnet, dass** die Auslagerungstemperatur mindestens 700 °C beträgt.

12. Verwendung oder Verfahren nach Anspruch 10 oder 11, **dadurch gekennzeichnet, dass** die Auslagerungsdauer 5 - 150 min beträgt.

## Claims

1. Use of a precipitation-hardening or solid-solution-strengthening, biocompatible cobalt-based alloy for the production of blanks which are provided so that, in the soft state thereof, material-removing machining can be carried out in order to carve implants or prostheses out of said blanks, which implants or prostheses are subjected to a heat treatment after the material-removing machining in order to adjust the final hardness of the implants or prostheses by means of precipitation hardening or solid-solution strengthening, wherein the cobalt-based alloy consists of (in % by weight),
| | |
|---|---|
| C: | up to 0.1%, |
| Si: | 0.8 - 2.0%, |
| Cr: | 22 - 29%, |
| Mn: | up to 1%, |
| Ni: | up to 3%, |
| Fe: | up to 3%, |
| Ce: | up to 1%, |
| Ga: | up to 5%, |
| B: | up to 2.5% |
and in each case at least one of the hardening precipitations or solid-solution-forming elements from the group comprising "Mo, W, Nb, Al, Ti", wherein for the concentrations of these elements, where present, the following proportion applies
| | |
|---|---|
| Mo: | 3 - 9%, |
| W: | 3 - 9%, |
| Nb | 3 - 9%, |
| Al: | 0.1 - 6 %, |
| Ti: | 0.1 - 6 %, |
and as residue Co and unavoidable impurities resulting from production.

2. Method for producing an implant or a prosthesis, comprising the following work steps:
- providing a soft blank, which consists of a precipitation-hardening or solid-solution-strengthening, biocompatible cobalt-based alloy,
- carving an implant or a prosthesis out of the soft blank by means of material-removing machining,
- heat-treating the implant or the prosthesis to adjust the hardness by means of precipitation hardening or solid-solution strengthening,
wherein the cobalt-based alloy consists of (in % by weight),
| | |
|---|---|
| C: | up to 0.1%, |
| Si: | 0.8 - 2.0%, |
| Cr: | 22 - 29%, |
| Mn: | up to 1%, |
| Ni: | up to 3%, |
| Fe: | up to 3%, |
| Ce: | up to 1%, |
| Ga: | up to 5%, |
| B: | up to 2.5% |
and in each case at least one of the hardening precipitations or solid-solution-forming elements from the group comprising "Mo, W, Nb, Al, Ti", wherein for the concentrations of these elements, where present, the following proportion applies
| | |
|---|---|
| Mo: | 3 - 9%, |
| W: | 3 - 9%, |
| Nb | 3 - 9%, |
| Al: | 0.1 - 6%, |
| Ti: | 0.1 - 6%, |
and as residue Co and unavoidable impurities resulting from production.

3. Use or method according to Claim 1 or 2, **characterised in that** the blanks are discs from which the respective implant or the respective prosthesis is carved by means of milling.

4. Use or method according to any one of the preceding claims, **characterised in that** the Cr concentration of the cobalt-based alloy is 23-27% by weight.

5. Use or method according to any one of the preceding claims, **characterised in that** for the concentrations of the hardening precipitations or solid-solution-forming elements present in the cobalt-based alloy the following proportion applies (in % by weight):
| | |
|---|---|
| Mo: | 4 - 6%, |
| W: | 4 - 6%, |
| Nb: | 4 - 6%, |
| Al: | 2 - 4%, |
| Ti: | 1 - 3%. |

6. Use or method according to any one of the preceding claims, **characterised in that** (in % by weight) the Mn concentration of the cobalt-based alloy is at most 0.5%, or the Ni concentration of the cobalt-based alloy is at most 0.1%.

7. Use or method according to any one of the preceding claims, **characterised in that** in each case at least two elements forming hardening precipitations are present in the cobalt-based alloy.

8. Use or method according to any one of the preceding claims, **characterised in that** the blanks are solution-annealed before the material-removing machining thereof at a temperature of from 1050 - 1300°C over a period of from 15 - 600 min.

9. Use or method according to Claim 8, **characterised in that** after the solution-annealing the blanks are cooled to room temperature at a cooling speed of from 5 - 1000 K/min.

10. Use or method according to any one of the preceding claims, **characterised in that** the implants or prostheses are kept at an ageing temperature of from 600 - 1000°C for an ageing period of at least 5 min and at most 600 min in order to adjust the hardness thereof.

11. Use or method according to Claim 10, **characterised in that** the ageing temperature is at least 700°C.

12. Use or method according to Claim 10 or 11, **characterised in that** the ageing period is from 5 - 150 min.

## Revendications

1. Utilisation d'un alliage biocompatible à base de cobalt, se durcissant par précipitation ou par formation de cristaux mixtes, pour fabriquer des ébauches, lesquelles sont prévues afin de réaliser à partir de celles-ci, à l'état mou, des implants ou des prothèses par usinage par enlèvement de matière, ces implants ou prothèses étant soumis, après l'usinage par enlèvement de matière, à un traitement thermique afin d'ajuster la dureté finale des implants ou des prothèses par durcissement par précipitation ou par formation de cristaux mixtes, l'alliage à base de cobalt se composant de (en % en poids)
C : jusqu'à 0,1 %,
Si : 0,8 - 2,0 %
Cr : 22 - 29 %,
Mn : jusqu'à 1 %,
Ni : jusqu'à 3 %,
Fe : jusqu'à 3 %,
Ce : jusqu'à 1 %,
Ga : jusqu'à 5 %,
B : jusqu'à 2,5 %
ainsi que d'au moins respectivement un des précipités accroissant la dureté ou éléments formant les cristaux mixtes choisi parmi le groupe « Mo, W, Nb, AI, Ti », où pour les teneurs de ces éléments, s'ils sont présents, les conditions suivantes s'appliquent
Mo : 3 - 9 %,
W : 3 - 9 %,
Nb : 3 - 9 %,
Al : 0,1 - 6 %,
Ti : 0,1 - 6 %,
et le reste étant du Co et des impuretés inévitables liées à la fabrication.

2. Procédé de fabrication d'un implant ou d'une prothèse, comportant les étapes de travail suivantes :
- Mise à disposition d'une ébauche molle qui se compose d'un alliage biocompatible à base de cobalt se durcissant par précipitation ou par formation de cristaux mixtes,
- Réalisation d'un implant ou d'une prothèse à partir de l'ébauche molle par usinage par enlèvement de matière,
- Traitement thermique de l'implant ou de la prothèse pour l'ajustage de la dureté par durcissement par précipitation ou par formation de cristaux mixtes,
l'alliage à base de cobalt se composant de (en % en poids)
C : jusqu'à 0,1 %,
Si : 0,8 - 2,0 %
Cr : 22 - 29 %,
Mn : jusqu'à 1 %,
Ni : jusqu'à 3 %,
Fe : jusqu'à 3 %,
Ce : jusqu'à 1 %,
Ga : jusqu'à 5 %,
B : jusqu'à 2,5 %
ainsi que d'au moins respectivement un des précipités accroissant la dureté ou éléments formant les cristaux mixtes choisi parmi le groupe composé de « Mo, W, Nb, AI, Ti », où pour les teneurs de ces éléments, s'ils sont présents, les conditions suivantes s'appliquent
Mo : 3 - 9 %,
W : 3 - 9 %,
Nb : 3 - 9 %,
Al : 0,1 - 6 %,
Ti : 0,1 - 6 %,
et le reste étant du Co et des impuretés inévitables liées à la fabrication.

3. Utilisation ou procédé selon la revendication 1 ou 2, caractérisé(e) en ce que les ébauches sont des rondelles, à partir desquelles l'implant respectif ou la prothèse respective est usiné(e) par fraisage.

4. Utilisation ou procédé selon l'une des revendications précédentes, caractérisé(e) en ce que la teneur en Cr de l'alliage à base de cobalt est de 23 - 27 en % en poids.

5. Utilisation ou procédé selon l'une des revendications précédentes, caractérisé(e) en ce que pour les teneurs du ou des précipités ou bien de l'élément ou des éléments formant les cristaux mixtes accroissant la dureté ou présents respectivement dans l'alliage à base de cobalt, les conditions suivantes s'appliquent (en % en poids)
Mo : 4 - 6 %,
W : 4 - 6 %,
Nb : 4 - 6 %,
Al : 2 - 4 %,
Ti : 1 - 3 %.

6. Utilisation ou procédé selon l'une des revendications précédentes, caractérisé(e) en ce que la teneur (en % en poids) en Mn de l'alliage à base de cobalt est au maximum de 0,5 % ou la teneur (en % en poids) en Ni de l'alliage à base de cobalt est au maximum de 0,1 %.

7. Utilisation ou procédé selon l'une des revendications précédentes, caractérisé(e) en ce que respectivement au moins deux éléments formant des précipités accroissant la dureté sont présents dans l'alliage à base de cobalt.

8. Utilisation ou procédé selon l'une des revendications précédentes, caractérisé(e) en ce que les ébauches sont, avant leur usinage par enlèvement de matière, recuites par mise en solution à une température de 1 050 - 1 300 °C pendant une durée de 15 - 600 min.

9. Utilisation ou procédé selon la revendication 8, caractérisé(e) en ce que les ébauches sont, après le recuit par mise en solution, refroidies avec une vitesse de refroidissement de 5 - 1 000 K/min jusqu'à température ambiante.

10. Utilisation ou procédé selon l'une des revendications précédentes, caractérisé(e) en ce que les implants ou les prothèses sont maintenu(e)s, pour l'ajustage de leur dureté, pendant un temps de recuit d'au moins 5 min et tout au plus de 600 min à une température de recuit de 600 - 1 000 °C.

11. Utilisation ou procédé selon la revendication 10, caractérisé(e) en ce que la température de recuit est au moins de 700 °C.

12. Utilisation ou procédé selon la revendication 10 ou 11, caractérisé(e) en ce que la durée de recuit est de 5 - 150 min.
